# EUROPEAN PATENT APPLICATION

(11) **EP 4 613 482 A1**
(43) Date of publication of application: **10.09.2025**
(21) Application number: 25160008.6
(22) Date of filing: 25.02.2025
(51) Int. Cl.: B32B 37/00, A61F 13/15, B29C 65/08, B32B 37/04, B32B 38/00, D06J 1/00

(54) **A PREPARATION METHOD OF COMPOSITE NON-WOVEN FABRIC USING WRINKLES TO INCREASE ELASTICITY**

(30) Priority: 06.03.2024 CN 202410254445
(71) Applicant: Nitto Advanced Components Taicang Co., Ltd., Suzhou Taicang City, Jiangsu Province 215434 (CN)
(72) Inventor: Choi, Hong Lim, Seoul (KR); Lin, Bao Hui, Taicang Jiangsu (CN)
(74) Representative: Andrejewski - Honke Patent- und Rechtsanwälte Partnerschaft mbB

(57) **Abstract**

Disclosed is a preparation method of a composite non-woven fabric by pleating to enhance stretchability, including: S1: unwinding an upper layer of non-woven fabric and a stretch film, and adjusting them in position; S2: pleating the adjusted upper layer of non-woven fabric along a cross direction (CD) or machine direction (MD) through an upper pleating mechanism, and aligning the pleated upper layer of non-woven fabric with the stretch film; S3: feeding the upper layer of non-woven fabric and the stretch film aligned with each other into a first laminating device, and laminating a contact surface between the bottom of the pleat and the stretch film to obtain a double-layer stretch non-woven fabric material; and S4: rewinding the laminated stretch material after tension adjustment and alignment. The present invention can produce non only a CD- or MD-stretch material but also a four-way stretch material; by adding a pleated design to the non-woven fabric, it can decrease or even replace stretching of the stretch film and reduce the probability of film breakage; moreover, it can realize local stretchability and improve connection firmness of the stretch non-woven fabric by a laminating means of layered welding.

## Description

### TECHNICAL FIELD

The present invention relates to the technical field of stretch materials, and in particular, to a preparation method of a composite non-woven fabric by pleating to enhance stretchability.

### BACKGROUND

Conventional and commercially available stretch non-woven fabric materials in the prior art are prepared by adding a stretch film stretched along a CD or MD between an upper layer of non-woven fabric and a lower layer of non-woven fabric and laminating the three layers of materials together through ultrasonic welding. The laminated material has stretchability along the CD or MD; however, the preparation process has some shortcomings: first, it can only achieve unidirectional stretchability; second, the preparation process poses extremely high stretch rate and quality requirements for the stretch film, resulting in a high scrap rate and frequent film breaks; and third, there is no way to achieve local release of stretchability.

Therefore, in view of the shortcomings in the prior art, it is necessary to design a preparation method of a composite non-woven fabric by pleating to enhance stretchability to solve the above problems.

It should be noted that the above description of the background is only intended to facilitate a clear and complete description of the technical solutions of the present invention and to help those skilled in the art understand the present invention. It does not mean that the above content is known to those skilled in the art simply because it is set forth in the background of the present invention.

### SUMMARY

In order to overcome the shortcomings in the prior art, the present invention discloses a preparation method of a composite non-woven fabric by pleating to enhance stretchability, which can not only decrease or even replace stretching of a stretch film by adding a pleated design to the non-woven fabric but also enable a stretch non-woven fabric material with four-way stretchability through a design of intersection between the pleating direction and the stretch direction of the stretch film and with good air permeability and high welding strength by optimizing the welding spot.

The present invention discloses a preparation method of a composite non-woven fabric by pleating to enhance stretchability, including the following steps:
S1: unwinding an upper layer of non-woven fabric and a stretch film, and adjusting them in position;
S2: pleating the adjusted upper layer of non-woven fabric uniformly along a cross direction (CD) or machine direction (MD) through an upper pleating mechanism, and correcting and aligning the pleated upper layer of non-woven fabric with the stretch film;
S3: feeding the upper layer of non-woven fabric and the stretch film corrected and aligned with each other into a first laminating device, and laminating a contact surface between the bottom of the pleat and the stretch film through the first laminating device to obtain a double-layer stretch non-woven fabric material, thereby avoiding damage to the stretch film during stretching and welding by replacing stretching of the stretch film with pleating; and
S4: rewinding the laminated stretch material after tension adjustment and alignment.

In a preferred technical solution, in the step S2, when the upper layer of non-woven fabric is uniformly pleated along the CD, the stretch film is stretched along the MD; and when the upper layer of non-woven fabric is uniformly pleated along the MD, the stretch film is stretched along the CD, thereby enabling the stretch non-woven fabric material with four-way stretchability through a design of intersection.

In a preferred technical solution, in the step S1, the stretch film is first slit into strips as required after unwinding, and then in the step S2, the upper layer of non-woven fabric is matched with the stretch film after being slit into strips and locally pleated along the CD. The width of the non-woven fabric in the locally pleated area is between 0.3 and 0.7 times that before local pleating, thereby obtaining a non-woven fabric material with local stretchability.

In a preferred technical solution, in the step S2, when the upper layer of non-woven fabric is uniformly pleated along the CD, the stretch film is stretched along the CD; and when the upper layer of non-woven fabric is uniformly pleated along the MD, the stretch film is stretched along the MD, thereby obtaining a highly CD- or MD-stretch non-woven fabric material.

In a preferred technical solution, the step S3 further includes: adding a lower layer of non-woven fabric that is consistent with the upper layer of non-woven fabric in pleating direction and position under the obtained double-layer stretch non-woven fabric material, correcting and aligning the double-layer stretch non-woven fabric material with the lower layer of non-woven fabric and feeding them into a second laminating device, and laminating a contact surface between the bottom of the pleat of the lower layer of non-woven fabric and the stretch film through the second laminating device to obtain a three-layer stretch non-woven fabric material. The layered lamination operation can make the upper and lower layers of non-woven fabrics more firmly connected and difficult to be torn apart.

In a preferred technical solution, the first laminating device and the second laminating device are both hot-melt laminating devices; the hot-melt laminating devices consist of a bottom roller and a hot melting unit; the bottom roller is provided with a welding pattern structure for forming a uniform welding spot on the contact surface between the bottom of the pleat and the stretch film; during a hot-melt lamination operation, in combination with a tension-adjusting device, a front side of the welding spot is melt and torn apart from the stretch film under an action of tension to form a pore; and the size of the pore is adjusted through the distances before and after the welding spot.

In a preferred technical solution, the hot melting unit can employ ultrasonic welding or thermocompression welding, and the welding pattern structure is a uniformly distributed square welding spot or a uniformly distributed circular or oval welding spot in a dislocated manner; for the uniformly distributed square welding spot, the stretchability can be enhanced by increasing a ratio of length to width of the welding spot in a stretch direction while ensuring air permeability; and for the uniformly distributed circular or oval welding spot in the dislocated manner, the stretchability can be enhanced by increasing a ratio of a distance between adjacent columns of welding spots to a distance between adjacent rows of welding spots in the stretch direction while ensuring air permeability, thereby enhancing stretchability while maintaining connection strength and air permeability.

In a preferred technical solution, a longitudinal distance a₁ between the square welding spots is 0.5-5 times a transverse length c₁ of the square welding spot, a transverse distance b₁ between the square welding spots is 0.5-3 times the transverse length c₁ of the square welding spot, and a longitudinal width d₁ of the square welding spot is 0.1-2 times the transverse length c₁ thereof, where c₁ and d₁ determine the area of a single welding spot; b₁ and a₁ determine the number of welding spots per unit area; the higher the proportion of welding spot area per unit area, the better the air permeability, but the strength will decrease to some extent, so the above ratios can ensure that the strength and air permeability of the connection are within an appropriate range; and meanwhile, the ratio of c₁ to d₁ determines the stretchability of the material along the MD and CD: when the ratio is greater than 1, the stretchability along the MD is greater than that along the CD, and when the ratio is less than 1, the stretchability along the MD is less than that along the CD. A longitudinal distance a₂ between the circular or oval welding spots is 1-5 times a longitudinal axis length c₂ of the circular or oval welding spot, a transverse distance b₂ between the circular or oval welding spots is 1-5 times the longitudinal axis length c₂ of the circular or oval welding spot, a transverse axis length d₂ of the circular or oval welding spot is 0.5-5 times the longitudinal axis length c₂ thereof, and straight-line distances e and f between the welding spots in the dislocated manner are 1-10 times the longitudinal axis length c₂ of the welding spot in the dislocated manner, where c₂ and d₂ determine the area of a single welding spot; b₂ and a₂ determine the number of welding spots per unit area; the higher the proportion of welding spot area per unit area, the better the air permeability, but the strength will decrease to some extent, so the above ratios can ensure that the strength and air permeability of the connection are within an appropriate range; and meanwhile, the ratio of 2b₂ to a₂ determines the stretchability of the material along the MD and CD: when the ratio is greater than 1, the stretchability along the MD is greater than that along the CD, and when the ratio is less than 1, the stretchability along the MD is less than that along the CD.

In a preferred technical solution, the upper layer of non-woven fabric and the lower layer of non-woven fabric can be selected from spunbond non-woven fabrics, hot air-through non-woven fabrics, hot-rolled non-woven fabrics, and highly stretch non-woven fabrics; and the stretch film can be selected from PE films, PET films, SBS films, and POE films.

In a preferred technical solution, when the stretch film is stretched along the MD, a stretch ratio ranges between 1.5 and 5 times, and a shrink ratio of the stretch film along the CD ranges between 0.5 and 0.9 times; and when the stretch film is stretched along the CD, a stretch ratio ranges between 1.5 and 3 times, and a shrink ratio of the stretch film along the MD ranges between 1 and 1.5 times.

By applying the above technical solutions, the preparation method of the composite non-woven fabric by pleating to enhance stretchability according to the present invention has the following beneficial effects:
(1) it can easily realize the production of not only CD- or MD-stretch materials but also four-way stretch materials;
(2) by adding a pleated design to the non-woven fabric, it can decrease or even replace stretching of the stretch film, thereby greatly reducing the probability of film breakage;
(3) by combining the strip-shaped stretch film with the local pleating design, it can achieve local stretchability on the surface of the material; and
(4) by using a laminating means of layered welding, it can enhance the connection firmness of the stretch non-woven fabric.

### BRIEF DESCRIPTION OF THE DRAWINGS

FIG. 1 is a transverse cross-sectional view of a double-layer stretch composite non-woven fabric according to the present invention;
FIG. 2 is a transverse cross-sectional view of a three-layer stretch composite non-woven fabric according to the present invention;
FIG. 3 is a schematic diagram of a preparation process of a double-layer stretch composite non-woven fabric according to the present invention;
FIG. 4 is a schematic diagram of a preparation process of a three-layer stretch composite non-woven fabric according to the present invention;
FIG. 5 is a schematic diagram of changes in size of a non-woven fabric when it is pleated along a CD according to the present invention;
FIG. 6 is a schematic diagram of changes in size of a stretch film when it is stretched along a CD according to the present invention;
FIG. 7 is a schematic diagram of changes in size of a local pleat of a non-woven fabric according to the present invention;
FIG. 8 is a schematic diagram of a welding pattern structure of a square welding spot according to the present invention; and
FIG. 9 is a schematic diagram of a welding pattern structure of a circular or oval welding spot according to the present invention.

### DETAILED DESCRIPTION OF THE EMBODIMENTS

The following illustrates the implementation manners of the present invention through embodiments, and those skilled in the art can easily understand other advantages and beneficial effects of the present invention from the content disclosed herein.

In the description of the present invention, it should be noted that the orientations or positional relationships indicated by the terms "center", "upper", "lower", "left", "right", "vertical", "horizontal", "inside", "outside", and the like are based on those shown in the accompanying drawings or the orientations or positional relationships in which the invented product is usually placed in use, and intended only for the convenience of describing the present invention and simplifying the description rather than for indicating or implying that the referred devices or elements must be provided with a particular orientation or constructed or operated in a particular orientation; therefore, these terms should not be construed as limiting the present invention. Furthermore, the terms "first", "second", "third", and the like are used for descriptive purposes only and should not be construed as indicating or implying relative importance. The terms "horizontal", "vertical", "overhanging", and the like do not mean that the referred component is required to be absolutely horizontal or overhanging, but can be slightly tilted. For example, "horizontal" only means that the direction is more horizontal relative to a "vertical" direction, and does not mean that the structure must be absolutely horizontal, but can be slightly tilted.

### Embodiment 1:

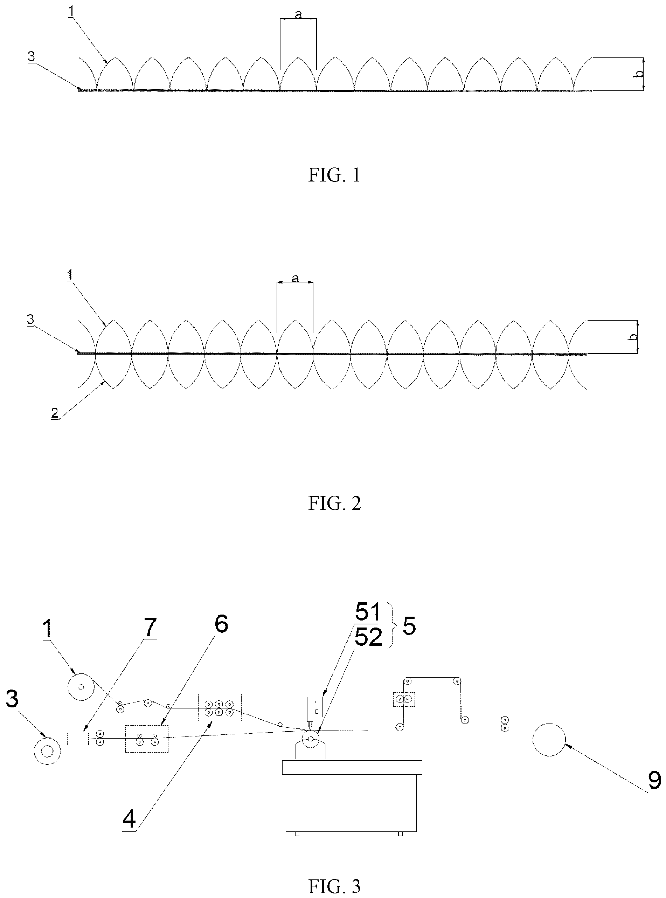

As shown in FIG. 3, an upper layer of non-woven fabric 1 and a stretch film 3 were first unwound through an unwinding machine and then adjusted in position through a buffer tower, a driving roller, a tension control unit, and a deflection corrector, where the upper layer of non-woven fabric 1 was a spunbond non-woven fabric and the stretch film 3 was a PE film.

As shown in FIGs. 1, 3, and 5, the adjusted upper layer of non-woven fabric 1 was uniformly pleated along a CD through a pleating mechanism 4, with the width a of the pleat being between 0.5-4 mm and the height b thereof being between 0.3-2 mm; the transverse shrink ratio after pleating ranged between 0.4 and 0.9 times; the stretch film 3 was not stretched; and the pleated upper layer of non-woven fabric 1 and the stretch film 3 were corrected and aligned with each other, where the shrink ratio = post-pleating size d / pre-pleating size D.

As shown in FIGs. 1, 3, and 8, the upper layer of non-woven fabric 1 and the stretch film 3 after being corrected and aligned with each other were fed into a first laminating device 5 and laminated through a first ultrasonic welding unit 51 and a first bottom roller 52, with uniformly distributed square welding spots formed on a contact surface between the bottom of the pleat and the stretch film, and in combination with a tension-adjusting device, a front side of the welding spot was melt and torn apart from the stretch film under an action of tension to form a pore. Finally, a CD-stretch non-woven fabric material was obtained, where the sizes of the welding spots were 0.5%c₁ ≤ a₁ ≤ 5*c₁, 0.5*c₁ ≤ b₁ ≤ 3*c₁, and 0.1*c₁ ≤ d₁ ≤ 2*c₁ respectively.

As shown in FIG 3, the laminated stretch material passed through the tension control unit, the driving roller, and the deflection corrector, and finally entered a rewinding machine 9 for rewinding.

### Embodiment 2:

As shown in FIG. 3, an upper layer of non-woven fabric 1 and a stretch film 3 were first unwound through an unwinding machine and then adjusted through a buffer tower, a driving roller, a tension control unit, and a deflection corrector.

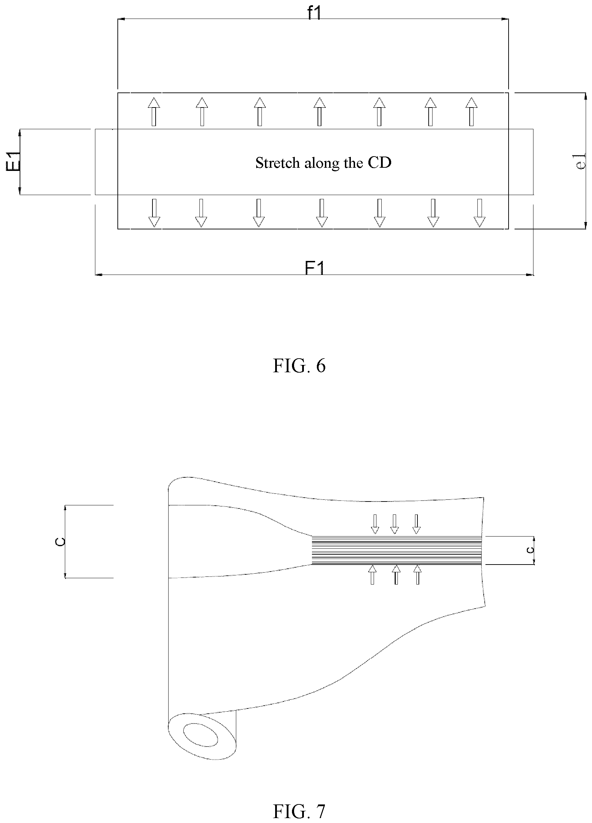

As shown in FIGs. 1, 3, and 6, the adjusted upper layer of non-woven fabric 1 was uniformly pleated along an MD through a pleating mechanism 4, with the width a of the pleat being between 0.5-4 mm and the height b thereof being between 0.3-2 mm; the stretch film 3 was stretched along a CD through a stretch film stretching device 6, with the stretch ratio of the stretch film along the CD ranging between 1.5-3 times and the transverse shrink ratio of the stretch film 3 ranging between 1-1.5 times; and the pleated upper layer of non-woven fabric 1 and the stretch film 3 were corrected and aligned with each other, where the stretch ratio of the stretch film 3 = post-stretching width e1 / pre-stretching width E1 and the shrink ratio of the stretch film 3 = post-stretching length f1 / pre-stretching length F1 when the stretch film was stretched along the CD.

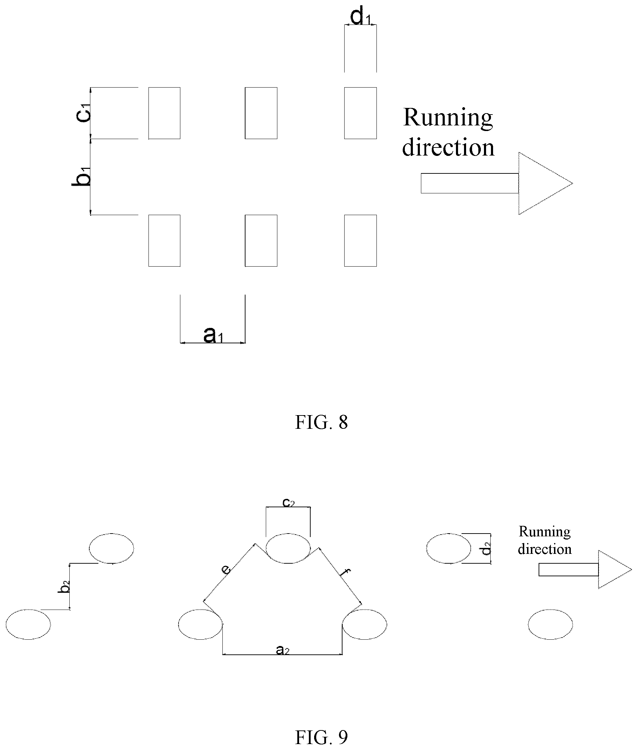

As shown in FIGs. 3 and 9, the upper layer of non-woven fabric 1 and the stretch film 3 after being corrected and aligned with each other were fed into a first laminating device 5 and laminated through a first ultrasonic welding unit 51 and a first bottom roller 52, with uniformly distributed oval welding spots formed in a dislocated manner on a contact surface between the bottom of the pleat and the stretch film 3, and in combination with a tension-adjusting device, a front side of the welding spot was melt and torn apart from the stretch film under an action of tension to form a pore. Finally, a four-way stretch material was obtained, where the sizes of the welding spots were 1*c₂ ≤ a₂ ≤ 5*c₂, 1*c₂ ≤ b₂ ≤ 5*c₂, 0.5*c₂ ≤ d₂ ≤ 5*c₂, 1 *c₂≤ e ≤ 10*c₂, and 1*c₂ ≤ f ≤ 10*c₂ respectively.

As shown in FIG. 3, the laminated stretch material passed through the tension control unit, the driving roller, and the deflection corrector, and finally entered a rewinding machine 9 for rewinding.

The obtained four-way stretch material was tested, with the test results shown in Table 1:

| Test Item | Test Method | Unit | Sample 1 | Sample 2 | Sample 3 |
|---|---|---|---|---|---|
| Basis weight | ASTM D5035 | g/m² | 77.3 | 78.5 | 79.1 |
| Tensile strength along the MD | ASTM D3776 | N/2Inch | 51.2 | 59.9 | 65.3 |
| Elongation at break along the MD | ASTM D3776 | % | 457.5 | 436.5 | 392.1 |
| Tensile strength along the CD | ASTM D3776 | N/2Inch | 21.7 | 25.8 | 23.0 |
| Elongation at break along the CD | ASTM D3776 | % | 349.5 | 436.1 | 415.0 |
| Air permeability | ISO 9237 | mm/s | 355 | 321 | 360 |

### Embodiment 3:

As shown in FIG. 3, an upper layer of non-woven fabric 1 and a stretch film 3 were first unwound through an unwinding machine, and then the upper layer of non-woven fabric 1 was adjusted through a buffer tower, a driving roller, a tension control unit, and a deflection corrector while the stretch film 3 was slit into strips as required using a slitting device 7 and then adjusted through the buffer tower, the driving roller, the tension control unit, and the deflection corrector.

As shown in FIGs. 1, 3, and 7, the upper layer of non-woven fabric 1 was matched with the stretch film after being slit into strips as required and locally pleated along a CD through a pleating mechanism 4, with the width a of the pleat being between 0.5-4 mm and the height b thereof being between 0.3-2 mm; the shrink ratio of the non-woven fabric in the locally pleated area ranged between 0.3-0.7 times; the stretch film 3 was not stretched; and the pleated upper layer of non-woven fabric 1 and the stretch film 3 were corrected and aligned with each other, where the shrink ratio of the locally pleated area = post-local pleating size c / pre-local pleating size C.

As shown in FIG. 3, the upper layer of non-woven fabric 1 and the stretch film 3 after being corrected and aligned with each other were fed into a first laminating device 5 and laminated through a first ultrasonic welding unit 51 and a first bottom roller 52, and in combination with a tension-adjusting device, a front side of the welding spot was melt and torn apart from the stretch film under an action of tension to form a pore. Finally, a locally strip-shaped CD-stretch material was obtained.

As shown in FIG. 3, the laminated stretch material passed through the tension control unit, the driving roller, and the deflection corrector, and finally entered a rewinding machine 9 for rewinding.

The obtained locally stretch material was tested, with the test results shown in Table 2:

| Test Item | Test Method | Unit | Sample 1 | Sample 2 | Sample 3 |
|---|---|---|---|---|---|
| Tensile strength along the CD | ASTM D3776 | N/2Inch | 23.7 | 22.8 | 26.0 |
| Elongation at break along the CD | ASTM D3776 | % | 301.4 | 295.7 | 310.7 |

### Embodiment 4:

As shown in FIG. 3, an upper layer of non-woven fabric 1 and a stretch film 3 were first unwound through an unwinding machine and then adjusted through a buffer tower, a driving roller, a tension control unit, and a deflection corrector.

As shown in FIGs. 1 and 3, the adjusted upper layer of non-woven fabric 1 was uniformly pleated along a CD through a pleating mechanism 4, with the width a of the pleat being between 0.5-4 mm and the height b thereof being between 0.3-2 mm; the stretch film 3 was stretched along the CD through a stretch film stretching device 6; and the pleated upper layer of non-woven fabric 1 and the stretch film 3 were corrected and aligned with each other.

As shown in FIG. 3, the upper layer of non-woven fabric 1 and the stretch film 3 after being corrected and aligned with each other were fed into a first laminating device 5 and laminated through a first ultrasonic welding unit 51 and a first bottom roller 52, and in combination with a tension-adjusting device, a front side of the welding spot was melt and torn apart from the stretch film under an action of tension to form a pore. Finally, a highly CD-stretch material was obtained.

As shown in FIG. 3, the laminated stretch material passed through the tension control unit, the driving roller, and the deflection corrector, and finally entered a rewinding machine 9 for rewinding.

### Embodiment 5:

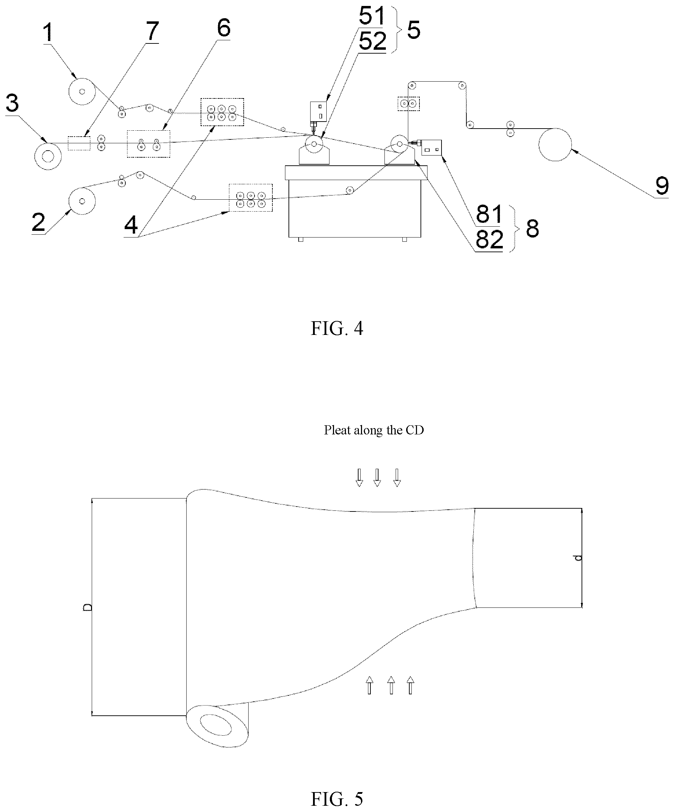

As shown in FIG. 4, an upper layer of non-woven fabric 1, a stretch film 3, and a lower layer of non-woven fabric 2 were first unwound through an unwinding machine and then adjusted through a buffer tower, a driving roller, a tension control unit, and a deflection corrector.

As shown in FIGs. 2 and 4, the upper layer of non-woven fabric 1 and the lower layer of non-woven fabric 2after being adjusted were uniformly pleated along an MD through a pleating mechanism 4; the stretch film 3 was stretched along a CD through a stretch film stretching device 6; the pleating directions of the upper layer of non-woven fabric 1 and the lower layer of non-woven fabric 2 in the corresponding positions were consistent; and the pleated upper layer of non-woven fabric 1 and the stretch film 3 were corrected and aligned with each other.

As shown in FIG. 4, the upper layer of non-woven fabric 1 and the stretch film 3 after being corrected and aligned with each other were fed into a first laminating device 5 and laminated through a first ultrasonic welding unit 51 and a first bottom roller 52, with uniformly distributed oval welding spots formed in a dislocated manner on a contact surface between the bottom of the pleat and the stretch film 3, and in combination with a tension-adjusting device, a front side of the welding spot was melt and torn apart from the middle layer of stretch film under an action of tension to form a pore. Then, a double-layer four-way stretch material was obtained. The obtained double-layer four-way stretch material and the pleated lower layer of non-woven fabric 2 were aligned and fed into a second laminating device 8, and laminated through a second ultrasonic welding unit 81 and a second bottom roller 82; and then, a contact surface between the bottom of the pleat of the lower layer of non-woven fabric 2 and the stretch film 3 was welded to obtain a three-layer four-way stretch non-woven fabric material.

As shown in FIG. 4, the laminated stretch material passed through the tension control unit, the driving roller, and the deflection corrector, and finally entered a rewinding machine 9 for rewinding.

To sum up, compared with the prior art, the present invention has the following advantages: it can produce non only a CD- or MD-stretch non-woven fabric material but also a four-way stretch non-woven fabric material; by adding a pleated design to the non-woven fabric, it can decrease or even replace stretching of the stretch film and reduce the probability of film breakage; moreover, it can produce a non-woven fabric material with local stretchability by using a strip-shaped stretch film in combination with a local pleating design.

The above embodiments are only intended to exemplarily illustrate the principle and effect of the present invention rather than to limit the present invention. Any person of ordinary skill in the art can modify or change the above embodiments without departing from the spirit and scope of the present invention. Therefore, all equivalent modifications or changes made by those of ordinary skill in the art without departing from the spirit and technical ideal disclosed by the present invention should still fall within the claims of the present invention.

## Claims

1. A preparation method of a composite non-woven fabric by pleating to enhance stretchability, wherein the preparation method comprises the following steps:
S1: unwinding an upper layer of non-woven fabric and a stretch film, and adjusting them in position;
S2: pleating the adjusted upper layer of non-woven fabric uniformly along a cross direction (CD) or machine direction (MD) through an upper pleating mechanism, and correcting and aligning the pleated upper layer of non-woven fabric with the stretch film;
S3: feeding the upper layer of non-woven fabric and the stretch film corrected and aligned with each other into a first laminating device, and laminating a contact surface between the bottom of the pleat and the stretch film through the first laminating device to obtain a double-layer stretch non-woven fabric material; and
S4: rewinding the laminated stretch material after tension adjustment and alignment.

2. The preparation method of the composite non-woven fabric by pleating to enhance stretchability according to claim 1, wherein in the step S2, when the upper layer of non-woven fabric is uniformly pleated along the CD, the stretch film is stretched along the MD; and when the upper layer of non-woven fabric is uniformly pleated along the MD, the stretch film is stretched along the CD.

3. The preparation method of the composite non-woven fabric by pleating to enhance stretchability according to claim 1, wherein in the step S1, the stretch film is first slit into strips as required after unwinding, and then in the step S2, the upper layer of non-woven fabric is matched with the stretch film after being slit into strips, and locally pleated along the CD.

4. The preparation method of the composite non-woven fabric by pleating to enhance stretchability according to claim 1, wherein in the step S2, when the upper layer of non-woven fabric is uniformly pleated along the CD, the stretch film is stretched along the CD; and when the upper layer of non-woven fabric is uniformly pleated along the MD, the stretch film is stretched along the MD.

5. The preparation method of the composite non-woven fabric by pleating to enhance stretchability according to any one of claims 1-4, wherein the step S3 further comprises: adding a lower layer of non-woven fabric that is consistent with the upper layer of non-woven fabric in pleating direction and position under the obtained double-layer stretch non-woven fabric material, correcting and aligning the double-layer stretch non-woven fabric material with the lower layer of non-woven fabric and feeding them into a second laminating device, and laminating a contact surface between the bottom of the pleat of the lower layer of non-woven fabric and the stretch film through the second laminating device to obtain a three-layer stretch non-woven fabric material.

6. The preparation method of the composite non-woven fabric by pleating to enhance stretchability according to claim 5, wherein the first laminating device and the second laminating device are both hot-melt laminating devices; the hot-melt laminating devices consist of a bottom roller and a hot melting unit; the bottom roller is provided with a welding pattern structure for forming a uniform welding spot on the contact surface between the bottom of the pleat and the stretch film; and during a hot-melt lamination operation, in combination with a tension-adjusting device, a front side of the welding spot is melt and torn apart from the stretch film under an action of tension to form a pore.

7. The preparation method of the composite non-woven fabric by pleating to enhance stretchability according to claim 6, wherein the hot melting unit can employ ultrasonic welding or thermocompression welding, and the welding pattern structure is a uniformly distributed square welding spot or a uniformly distributed circular or oval welding spot in a dislocated manner; for the uniformly distributed square welding spot, the stretchability can be enhanced by increasing a ratio of length to width of the welding spot in a stretch direction while ensuring air permeability; and for the uniformly distributed circular or oval welding spot in the dislocated manner, the stretchability can be enhanced by increasing a ratio of a distance between adjacent columns of welding spots to a distance between adjacent rows of welding spots in the stretch direction while ensuring air permeability.

8. The preparation method of the composite non-woven fabric by pleating to enhance stretchability according to claim 7, wherein a longitudinal distance a₁ between the square welding spots is 0.5-5 times a transverse length c₁ of the square welding spot, a transverse distance b₁ between the square welding spots is 0.5-3 times the transverse length c₁ of the square welding spot, and a longitudinal width d₁ of the square welding spot is 0.1-2 times the transverse length c₁ thereof; and a longitudinal distance a₂ between the circular or oval welding spots is 1-5 times a longitudinal axis length c₂ of the circular or oval welding spot, a transverse distance b₂ between the circular or oval welding spots is 1-5 times the longitudinal axis length c₂ of the square welding spot, a transverse axis length d₂ of the circular or oval welding spot is 0.5-5 times the longitudinal axis length c₂ thereof, and straight-line distances e and f between the welding spots in the dislocated manner are 1-10 times the longitudinal axis length c₂ of the welding spot in the dislocated manner.

9. The preparation method of the composite non-woven fabric by pleating to enhance stretchability according to claim 5, wherein the upper layer of non-woven fabric and the lower layer of non-woven fabric can be selected from spunbond non-woven fabrics, hot air-through non-woven fabrics, hot-rolled non-woven fabrics, and highly stretch non-woven fabrics; and the stretch film can be selected from PE films, PET films, SBS films, and POE films.

10. The preparation method of the composite non-woven fabric by pleating to enhance stretchability according to claim 2, wherein when the stretch film is stretched along the MD, a stretch ratio ranges between 1.5 and 5 times, and a shrink ratio of the stretch film along the CD ranges between 0.5 and 0.9 times; and when the stretch film is stretched along the CD, a stretch ratio ranges between 1.5 and 3 times, and a shrink ratio of the stretch film along the MD ranges between 1 and 1.5 times.
